# EUROPEAN PATENT SPECIFICATION

(11) **EP 3 561 066 B1**
(45) Date of publication and mention of the grant of the patent: **07.02.2024**
(21) Application number: 16924768.1
(22) Date of filing: 21.12.2016
(51) Int. Cl.: C12P 13/00, C12P 17/10, C05C 11/00, C05F 1/00, C05F 11/00, C05F 11/10

(54) **METHOD FOR OBTAINING POLYAMINES FROM A PROTEIN MATERIAL**
VERFAHREN ZUR GEWINNUNG VON POLYAMINEN AUS EINEM PROTEINMATERIAL
PROCÉDÉ D'OBTENTION DE POLYAMINES À PARTIR D'UNE MATIÈRE PROTÉIQUE

(43) Date of publication of application: 30.10.2019
(73) Proprietor: FERTINAGRO BIOTECH, S.L., 44195 Teruel (ES)
(72) Inventor: ATARES REAL, Sergio, 44195 Teruel (ES); ROMERO LOPEZ, Joaquin, 44195 Teruel (ES); SALAET MADORRAN, Ignasi, 44195 Teruel (ES); COLOM TOMAS, Elena, 44195 Teruel (ES); FERRER GINES, María, 44195 Teruel (ES); NARANJO OLIVERO, Miguel Angel, 44195 Teruel (ES)
(74) Representative: González López-Menchero, Álvaro Luis
(86) International application number: PCT/ES2016/070920
(87) International publication number: WO 2018/115538

(56) References cited:
- WO-A1-87/05895
- WO-A2-2014/049382
- DE-A1-102007 005 072
- ES-A1- 2 088 826
- US-A- 5 435 822
- US-A1- 2011 039 313
- WEICHAO MA ET AL: "Advances in Cadaverine Bacterial Production and Its Applications", ENGINEERING, vol. 3, no. 3, 1 June 2017 (2017-06-01), pages 308-317, XP055603579, ISSN: 2095-8099, DOI: 10.1016/J.ENG.2017.03.012
- KIM, B. et al.: "Biogenic amine formation and bacterial contribution in Natto products", FOOD CHEMISTRY, vol. 135, no. 3, 1 December 2012 (2012-12-01), pages 2005-2011, XP028935702,
- TRISTEZZA, M. et al.: "Biodiversity and safety aspects of yeast strain; characterized from vineyards and spontaneous fermentations in the Apulia Region, Italy", FOOD MICROBIOLOGY, vol. 36, no. 2, 1 December 2013 (2013-12-01), pages 335-342, XP028708752, DOI: doi:10.1016/j.fm.2013.07.001

## Description

The present invention relates to a method for obtaining polyamines from a protein material, in particular for obtaining biogenic polyamines, meaning polyamines formed by the transformation of the amino acids found in food by the effects of enzymes generated by microorganisms, specifically for obtaining histamine, putrescine, spermidine and cadaverine.

More specifically, the method of the invention enables these biogenic polyamines to be obtained from a biological material with a protein content of approximately 10% by weight by performing a hydrolysis process followed by fermenting wherein the microorganism *Bacillus subtilis* is used in order to convert the amino acids into these polyamines.

The method of the invention enables the biogenic polyamines to be obtained with a high concentration and in a reduced amount of time.

Polyamines are small molecules that are present in bacteria, plants and animal tissues, and they perform important biological functions, being essential in the processes of growth and differentiation of eukaryotic cells in living beings. Due to their biochemical characteristics they are involved in a large number of cellular processes, such as growth, plant development and, in certain situations, as a response to stress. For these reasons they can be catalogued as plant growth regulators (phytohormones). Because of the basic nature thereof, they interact with nucleic acids, with stabilising paper or as growth factors, thus, increasingly greater importance is attributed to them in cell proliferation.

The most eminent polyamines in plants are diamine putrescine, triamine spermidine and tetraamine spermine. Due to the polycationic nature thereof, they can bind and stabilise polymers rich in negative charges such as DNA (Prescott, L.M. (199). Microbiologia. McGraw-Hill Interamericana de España, S.A.U. ISBN 84-486-0261-7) but also phospholipids and proteins (Azcón-Bieto, J and Talón, M. (2000). Fundamentos de Fisiología Vegetal. Mc Graw Hill Interamericana de España SAU. ISBN 84-486-0258-7). Said functions are likewise postulated for animals and microorganisms. In animals, polyamines are derived from the metabolism of the amino acids or come from the metabolism of intestinal bacteria or from diet, entering the organism through enterohepatic circulation. In mammals, the most important examples of polyamines are spermine (four amino groups) and spermidine (three amino groups), both formed from the decarboxylation of ornithine, which forms an intermediary of two amino groups, the putrescine. Thus, in putrefaction in animals, putrescine accumulates, the ornithine loses CO₂, but the putrescine formed cannot be transformed into spermidine without the coenzyme AdoMet.

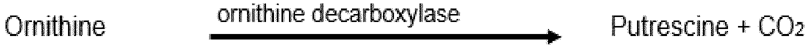

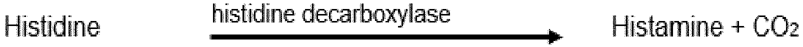

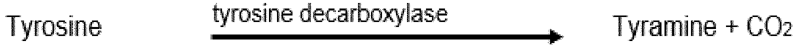

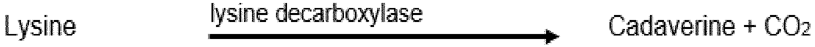

With these characteristics, polyamines influence cellular activity and a large number of physiological processes such as growth, plant development, biotic and abiotic stress (Guye et al., Polyamine titre in relation to chill-sensitivity in Phaseolus sp, Journal of Experimental Botany 1986 v. 37, p. 1036-1043,1986; Evans PT, Makmerg, R. Do polyamines have role in plant development? Annual Review of Plant Physiology and Molecular Biology, 1989. v. 87, p.519 -522; Faust M., Wang S.; Polyamines in horticulturally important plants. Horticultural Reviews, 1992 v.14, p. 333 - 356; Bais H.P., Ravishankar G.A, Role of polyamines in the ontogeny of plants and their biotechnological applications. Plant Cell Tissue and Organ Culture, 2002. v.69, p. 1-34).

They are found in all plant cells in three different states (Evans and Malmberg 1989 *supra*; Galston. A.W., Kaur-Shawney, R., Polyamines in plant physiology. Plant Physiology, 1990, v.94, p. 406 - 410):
- Free State, in which they are electrostatically active, i.e., they have the possibility of associating with negatively charged molecules (technically called anions).
- Soluble bond state, in which the polyamines form complexes with compounds having low molecular weight and are soluble in the cytoplasm of the cell and are protected against degradation during the transportation thereof through the plant.
- Insoluble bond state, when they are joined to macromolecules such as cellulose in the cell wall and anionic sites of the membranes. Due to the high molecular weight of this complex, the polyamines in this state do not occur in the cellular cytoplasm.

Thus, biogenic polyamines are aliphatic nitrogen compounds that are currently considered as regulators for the growth and development of plants because of the demonstrated effects thereof on growth, cell division and differentiation at low concentrations, for example, because of the polycationic nature thereof, they can bind to negatively charged molecules such as nucleic acids, proteins or phospholipids, altering the gene expression and the activity of certain enzymes, as well as varying the fluidity and permeability of the biological membranes. In any case, the polyamines act as a nitrogen reserve, constituting the only source of the same. The biosynthesis thereof is closely related to that of gaseous phytohormone ethylene, since S-adenosylmethionine is the common intermediary of both metabolic pathways. This distribution of S-adenosylmethionine can have significant physiological implications. The polyamines can be conjugated with hydroxycinnamic acids and perform functions, which are still unclear, in the processes of differentiation, flowering and ripening; moreover, they have an effect on the resistance to viruses and fungi in certain plants. The cell wall is one of the most important cellular compartments in relation to the degradative metabolism of polyamines, highlighting the increase in the conjugation of polyamines in the cell wall during cell aging (Gallardo, M., Matilla, A., Muñoz de Rueda, P. and Sanchez Calle, I.M. Department of Plant Biology. Universidad de Granada, Ars Pharm.37 (1), 17-27, (1996)).

Therefore, it would be desirable and it is an object of the invention to provide a method for obtaining these polyamines with a high concentration, good yield and in a short amount of time.

In document ES2088826 B1, "Method for obtaining putrescine and cadaverine diamines from treated natural products, the use thereof as an additive in fertilisers, and the corresponding fertiliser", it describes a method for obtaining putrescine and cadaverine diamines from treated natural products, with a mostly protein composition, which comprises performing a process of aerobic and/or anaerobic incubations of proteins, peptides and amino acids from treated natural products. The method essentially comprises a step of aerobic incubation with microorganisms facilitating the decarboxylation of certain amino acids, addition of proteases, homogenisation of the mixture and light aeration of the process, carrying out a control of the process with identification and quantification of the bacterial flora, and determining the concentration of the main active ingredients, especially putrescine and cadaverine, with a control of the pH and the temperature of the process and a second step of incubation in anaerobiosis with microorganisms that facilitate the decarboxylation of certain amino acids, the crop is homogenised again, and it is arranged in airless and hermetically sealed containers and finally a control step wherein the bacterial flora is identified and quantified, the concentrations of putrescine and cadaverine diamines are determined and the pH is controlled. In this document, it is indicated that the incubation times are variable, without being cited in any case. Likewise, in this document, the concentrations of polyamines obtained are not indicated and no reference is made to the yield of the process.

The objective of the invention is solved by providing a method for obtaining biogenic polyamines from a protein material, in particular histamine, putrescine, spermidine and cadaverine.

| | |
|---|---|
| 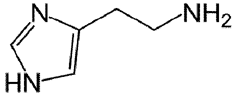 | Formula: C₅H₉N₃ |
| | Molecular mass: 111.06 g/mol |
| Histamine | CAS: 56-92-8 |
| 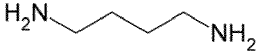 | Formula: C₄H₁₂N₂ |
| | Molecular mass: 88.15 g/mol |
| Putrescine | CAS: 110-60-1 |
| 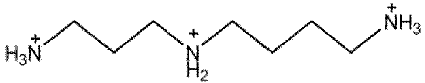 | Formula: C₇H₁₉N₃ |
| | Molecular mass: 145.25 g/mol |
| Spermidine | CAS: 124-2-92 |
| 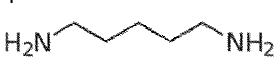 | Formula: C₅H₁₄N₂ |
| | Molecular mass: 102.178 g/mol |
| Cadaverine | CAS: 462-94-2 |

More specifically, the method of the invention enables these biogenic polyamines to be obtained from a protein material with a protein content of approximately 10% by weight essentially by performing a hydrolysis process followed by fermenting wherein the microorganism *Bacillus subtilis* is used in order to convert the amino acids of the protein material into these polyamines.

The method of the invention comprises the following steps:
1) A first step of performing hydrolysis of a protein material with a protein content of approximately 10%, followed by filtering to obtain a liquid permeate with a content of hydrolysed protein between 3 and 9%;
2) A second step of fermenting the permeate obtained previously by adding the microorganism *Bacillus subtilis* at a temperature of 25° to 35°C and keeping the pH between 5 and 7; and
3) Finally, a third step of purifying by means of filtering to eliminate the insoluble microorganisms and concentrating the obtained product by means of spray drying.

In reference to the protein material with a protein content of approximately 10%, it can be of animal origin, for example from blood and feathers from birds, or of plant origin, for example soy and yeast extracts, or combinations thereof.

This first step of hydrolysis of the protein material is carried out with 18% sulphuric acid at 130°C and a pressure of 1.5-2 bar and neutralisation with calcium hydroxide at a pH of 5-6.

The filtering of the hydrolysed material in the first step of the method is preferably performed by means of a filter press with a pore size between 150 and 300 micrometres in order to obtain a liquid permeate with the specified protein content.

This liquid permeate is taken directly to the fermentation step without there being a residence time in storage tanks, since in this way the permeate has a very low concentration of polluting organisms, especially if the hydrolysis conditions are the ones cited previously, which may interfere or otherwise affect the fermentation reaction.

In the fermentation step, added to the liquid permeate with a protein content of 3-9% is the microorganism *Bacillus subtilis,* preferably the *Bacillus subtilis* Strain: BS 161013 (deposited in the Spanish Type Culture Collection (CECT) with the reference number 9187 *Bacillus subtilis*)*.*

*Bacillus subtilis* is a species which promotes plant growth and one of the ways to carry out this function is by producing spermidine, which stimulates the roots of the plants. *Bacillus subtilis* is able to produce spermidine through two different pathways from arginine and also from methionine. During the degradation process of the arginine *Bacillus subtilis* also produces putrescine.
Species of the genus *Bacillus* are also capable of producing other polyamines by fermenting other amino acids. The preferred use of *Bacillus subtilis* CECT 9187 enables a high concentration of biogenic polyamines to be obtained in a relatively short amount of time compared to the use of other strains of this species.

In a preferred embodiment of the method, the temperature in the fermentation step 2) is maintained at 28°C, for which said fermentation is carried out in a reactor with temperature control with an accuracy of ± 0.1°C.

Since in this fermentation process the polyamines are produced in solution, the release of OH⁻ ions causes an increase in the pH, therefore it is necessary to control the pH in order to keep it within the indicated limits by adding sulphuric acid.

Thus, in another preferred embodiment of the method, the pH in the fermentation step 2) is maintained at 5.5, using a reactor with pH control of ± 0.1 to do so.

In a likewise preferred embodiment of the method of the invention, in step 2) nutrients are added to promote the growth of the inoculated microorganism, preferably selected from among yeast extract (0.1% -0.5%), sodium chloride (0.05%-0.1%), tween 80 (0.01%-0.08%), MgSO₄·7H₂O (0.01%-0.04%), MnSO₄·4H₂O (0.001%-0.01%) and FeSO₄·7H₂O (0.001%-0.008%), as well as a 1% extract of *Metschnikowia Pulcherrima,* and combinations thereof, percentages expressed by weight with respect to the weight of the fermentation broth.

Preferably, in step 3) purifying by means of filtering is carried out by means of the use of ceramic filters, in particular of 600 nm. The main advantage of using this type of filter is that it removes all types of microorganisms, for example, those which could harm the obtained product, performing a sterilisation by filtering. Likewise, this type of filter allows separation by sizes of up to 3,000 kDa and facilitates the removal of insoluble parts.

Once purified, the product is concentrated in this step 3). To do so, the water is removed in order to obtain a solid concentrate of polyamines with a content between 2 and 10%. This drying process is carried out by means of spray drying wherein the drying temperature must not exceed 400°C, since higher temperatures could degrade the final solid product obtained.

### Examples

Quantification of the biogenic amines was carried out by liquid chromatography with a fluorescence detector. For this, a Perkin Elmer chromatograph was used, with an automatic injector; UV-visible detector with readings at 254 nm and a C-18 reverse phase column (4.0 mm x 250 mm). The mobile phase was composed of a mixture of 0.01M potassium phosphate (2.15 grams in 1 l) and KCl in concentration of 0.5 mM (0.04 grams in 1 l). Injection volume from 15 µl to 25 µl. The applied flow rate is indicated below:

| Flow (ml) | T (min) | Phase A (%) | Phase B (%) |
|---|---|---|---|
| 0.2 | 6 | 0 | 100 |
| 1 | 10 | 50 | 50 |
| 1.5 | 16 | 100 | 0 |

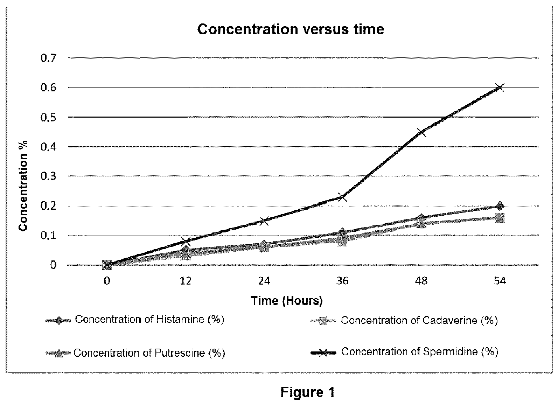

The results of the analyses are shown in the following table and in figure 1.

| Time (hours) | 0 | 12 | 24 | 36 | 48 | 54 |
|---|---|---|---|---|---|---|
| Concentration of Histamine (%) | 0 | 0.05 | 0.07 | 0.11 | 0.16 | 0.20 |
| Concentration of Cadaverine (%) | 0 | 0.03 | 0.06 | 0.08 | 0.14 | 0.16 |
| Concentration of Putrescine (%) | 0 | 0.04 | 0.06 | 0.09 | 0.14 | 0.16 |
| Concentration of Spermidine (%) | 0 | 0.08 | 0.15 | 0.23 | 0.45 | 0.60 |

The product was also quantified in terms of histamine, cadaverine, putrescine and spermidine polyamines obtained in the purification step before and after the concentration. The results are shown below:

| Before concentration | | Concentrated product | |
|---|---|---|---|
| | % | | % |
| Histamine | 0.01-0.25 | Histamine | 0.10-1.10 |
| Cadaverine | 0.01-0.20 | Cadaverine | 0.10-1.00 |
| Putrescine | 0.01-0.20 | Putrescine | 0.10-1.00 |
| Spermidine | 0.01-0.60 | Spermidine | 0.10-1.30 |
| | | Other polyamines* | 1.60-5.60 |
| * Not identified | | | |

For comparative purposes, the same process was carried out using the same initial materials and under the same conditions, according to the comparative process without previous digestion. The results obtained shown in the following table show that the concentration of polyamines is much lower than the one obtained by applying the process of the invention.

| Comparative process | | | |
|---|---|---|---|
| Before concentration | | Concentrated product | |
| | % | | % |
| Histamine | 0.01-0.10 | Histamine | 0.10-0.60 |
| Cadaverine | 0.01-0.10 | Cadaverine | 0.10-0.60 |
| Putrescine | 0.01-0.10 | Putrescine | 0.10-0.60 |
| Spermidine | 0.01-0.35 | Spermidine | 0.10-0.80 |
| | | Other polyamines* | 0.10-0.90 |
| * Not identified | | | |

## Claims

1. A method for obtaining biogenic polyamines from a protein material with a protein content of 10% by weight, wherein the process includes the following steps:
i. A first step of performing hydrolysis of a protein material with a protein content of 10%, wherein this hydrolysis of step i) is carried out with 18% sulphuric acid at 130°C and a pressure of 1.5-2 bar and neutralisation with calcium hydroxide at a pH of 5-6, followed by filtering to obtain a liquid permeate with a content of hydrolysed protein between 3 and 9%;
ii. A second step of fermenting the permeate obtained in i. by adding the microorganism Bacillus subtilis at a temperature of 25° to 35°C and keeping the pH between 5 and 7; and
iii. A third step of purifying by means of filtering to eliminate the insoluble microorganisms and concentrating the obtained product by means of spray drying.

2. The method for obtaining biogenic polyamines according to claim 1, **characterised in that** the protein material with a protein content of 10% is of animal origin, of plant origin or a combination of both.

3. The method for obtaining biogenic polyamines according to claim 1, **characterised in that** the filtering of the hydrolysed material in step i) is performed by means of a filter press with a pore size between 150 and 300 micrometres.

4. The method for obtaining biogenic polyamines according to claim 1, **characterised in that** in the fermentation step ii) the microorganism *Bacillus subtilis* belongs to the *Bacillus subtilis* strain CECT 9187.

5. The method for obtaining biogenic polyamines according to claim 1, **characterised in that** the temperature in the fermentation step ii) is kept at 28°C, for which said fermentation is carried out in a reactor with temperature control with an accuracy of ± 0.1°C.

6. The method for obtaining biogenic polyamines according to claim 1, **characterised in that** the pH in the fermentation step ii) is kept at 5.5.

7. The method for obtaining biogenic polyamines according to claim 1, **characterised in that** in step ii) nutrients are added to promote the growth of the inoculated microorganism selected from among yeast extract (0.1%-0.5%), sodium chloride (0.05%-0.1%), tween 80 (0.01%-0.08%), MgSO₄·7H₂O (0.01%-0.04%), MnSO₄·4H₂O (0.001%-0.01%) and FeSO₄·7H₂O (0.001%-0.008%), as well as a 1% extract of *Metschnikowia Pulcherrima,* and combinations thereof, percentages expressed by weight with respect to the weight of the fermentation broth.

8. The method for obtaining biogenic polyamines according to claim 1, **characterised in that** step iii) of purifying by filtering is carried out by the use of 600 nm ceramic filters.

9. The method for obtaining biogenic polyamines according to claim 1, **characterised in that** the concentration in step iii) is carried out by means of spray drying, wherein the drying temperature does not exceed 400°C.

10. The method for obtaining biogenic polyamines according to claim 1, **characterised in that** it enables a solid concentrate of polyamines to be obtained with a content between 2 and 10%.

11. The method for obtaining biogenic polyamines according to claim 10, **characterised in that** a solid concentrate is obtained which includes Histamine 0.10-1.10%, Cadaverine 0.10-1.00%, Putrescine 0.10-1.00%, Spermidine 0.10-1.30% and other polyamines 1.60-5.60%.

## Patentansprüche

1. Verfahren zur Gewinnung von biogenen Polyaminen aus einem Proteinmaterial mit einem Proteingehalt von 10 Gew.-%, wobei der Vorgang die folgenden Schritte einschließt:
i. Einen ersten Schritt zum Durchführen der Hydrolyse eines Proteinmaterials mit einem Proteingehalt von 10 %, wobei diese Hydrolyse von Schritt i) mit 18 %iger Schwefelsäure bei 130 °C und einem Druck von 1,5 bis 2 bar und Neutralisation mit Calciumhydroxid bei einem pH-Wert von 5-6 durchgeführt wird, gefolgt von Filtration, um ein flüssiges Permeat mit einem Gehalt an hydrolysiertem Protein zwischen 3 und 9 % zu gewinnen;
ii. Einen zweiten Schritt zum Fermentieren des in i. gewonnenen Permeats durch Zugabe des Mikroorganismus *Bacillus subtilis* bei einer Temperatur von 25° bis 35 °C und unter Beibehalten eines pH-Werts zwischen 5 und 7; und
iii. Einen dritten Schritt zum Reinigen durch Filtrieren, um die unlöslichen Mikroorganismen zu beseitigen, und Konzentrieren des gewonnenen Produkts durch Sprühtrocknung.

2. Verfahren zur Gewinnung von biogenen Polyaminen nach Anspruch 1, **dadurch gekennzeichnet, dass** das Proteinmaterial mit einem Proteingehalt von 10 % tierischen Ursprungs, pflanzlichen Ursprungs oder einer Kombination aus beiden ist.

3. Verfahren zur Gewinnung von biogenen Polyaminen nach Anspruch 1, **dadurch gekennzeichnet, dass** das Filtrieren des hydrolysierten Materials in Schritt i) mit Hilfe einer Filterpresse mit einer Porengröße zwischen 150 und 300 Mikrometern durchgeführt wird.

4. Verfahren zur Gewinnung von biogenen Polyaminen nach Anspruch 1, **dadurch gekennzeichnet, dass** im Schritt ii) der Fermentation der Mikroorganismus *Bacillus subtilis* zum *Bacillus subtilis* Stamm CECT 9187 gehört.

5. Verfahren zur Gewinnung von biogenen Polyaminen nach Anspruch 1, **dadurch gekennzeichnet, dass** die Temperatur im Schritt ii) der Fermentation auf 28 °C gehalten wird, wofür die Fermentation in einem Reaktor mit einer Temperatursteuerung mit einer Genauigkeit von ± 0,1 °C durchgeführt wird.

6. Verfahren zur Gewinnung von biogenen Polyaminen nach Anspruch 1, **dadurch gekennzeichnet, dass** der pH-Wert im Schritt ii) der Fermentation bei 5,5 gehalten wird.

7. Verfahren zur Gewinnung von biogenen Polyaminen nach Anspruch 1, **dadurch gekennzeichnet, dass** in Schritt ii) Nährstoffe zugegeben werden, um das Wachstum des inokulierten Mikroorganismus zu fördern, ausgewählt aus Hefeextrakt (0,1 %-0,5 %), Natriumchlorid (0,05 %-0,1 %), Tween 80 (0,01 %-0,08 %), MgSO₄·7H₂O (0,01 %- 0,04 %), MnSO₄·4H₂O (0,001 %-0,01 %) und FeSO₄·7H₂O (0,001 %-0,008 %) sowie einem 1 %igen Extrakt aus *Metschnikowia pulcherrima* und Kombinationen davon, wobei die Anteile in Gewichtsprozenten in Bezug auf das Gewicht der Fermentationsbrühe ausgedrückt sind.

8. Verfahren zur Gewinnung von biogenen Polyaminen nach Anspruch 1, **dadurch gekennzeichnet, dass** der Schritt iii) zum Reinigen durch Filtrieren unter Verwendung von Keramikfiltern mit 600 nm durchgeführt wird.

9. Verfahren zur Gewinnung von biogenen Polyaminen nach Anspruch 1, **dadurch gekennzeichnet, dass** die Aufkonzentrierung in Schritt iii) mittels Sprühtrocknung durchgeführt wird, wobei die Trocknungstemperatur 400 °C nicht überschreitet.

10. Verfahren zur Gewinnung von biogenen Polyaminen nach Anspruch 1, **dadurch gekennzeichnet, dass** es die Gewinnung eines festen Polyaminkonzentrats mit einem Gehalt zwischen 2 und 10 % ermöglicht.

11. Verfahren zur Gewinnung von biogenen Polyaminen nach Anspruch 10, **dadurch gekennzeichnet, dass** ein festes Konzentrat gewonnen wird, das Histamin 0,10-1,10 %, Cadaverin 0,10-1,00 %, Putrescin 0,10-1,00 %, Spermidin 0,10-1,30 % und andere Polyamine 1,60-5,60 % einschließt.

## Revendications

1. Procédé pour l'obtention de polyamines biogènes à partir d'une matière protéique ayant une teneur en protéines de 10 % en poids, le processus comprenant les étapes suivantes :
i. une première étape consistant à effectuer l'hydrolyse d'une matière protéique ayant une teneur en protéines de 10 %, cette hydrolyse de l'étape i) étant mise en oeuvre avec de l'acide sulfurique à 18 % à 130 °C et une pression de 1,5 à 2 bar et une neutralisation avec de l'hydroxyde de calcium à un pH de 5 à 6, suivie d'une filtration pour obtenir un perméat liquide ayant une teneur en protéines hydrolysées comprise entre 3 et 9 % ;
ii. une deuxième étape consistant à faire fermenter le perméat obtenu en i. par l'ajout du micro-organisme *Bacillus subtilis* à une température de 25 à 35 °C et le maintien du pH entre 5 et 7 ; et
iii. une troisième étape consistant à purifier au moyen d'une filtration pour éliminer les micro-organismes insolubles et d'une concentration du produit obtenu au moyen d'un séchage par pulvérisation.

2. Procédé pour l'obtention de polyamines biogènes selon la revendication 1, **caractérisé en ce que** la matière protéique ayant une teneur en protéines de 10 % est d'origine animale, d'origine végétale ou une combinaison des deux.

3. Procédé pour l'obtention de polyamines biogènes selon la revendication 1, **caractérisé en ce que** la filtration de la matière hydrolysée à l'étape i) est effectuée au moyen d'un filtre-presse ayant une taille des pores comprise entre 150 et 300 micromètres.

4. Procédé pour l'obtention de polyamines biogènes selon la revendication 1, **caractérisé en ce qu'**à l'étape de fermentation ii) le micro-organisme *Bacillus subtilis* appartient à la souche CECT 9187 de *Bacillus subtilis.*

5. Procédé pour l'obtention de polyamines biogènes selon la revendication 1, **caractérisé en ce que** la température à l'étape de fermentation ii) est maintenue à 28 °C, pour lequel ladite fermentation est mise en oeuvre dans un réacteur ayant une régulation de température avec une précision de ± 0,1 °C.

6. Procédé pour l'obtention de polyamines biogènes selon la revendication 1, **caractérisé en ce que** le pH à l'étape de fermentation ii) est maintenu à 5,5.

7. Procédé pour l'obtention de polyamines biogènes selon la revendication 1, **caractérisé en ce qu'**à l'étape ii) pour favoriser la croissance du micro-organisme inoculé on ajoute des nutriments choisis parmi de l'extrait de levure (0,1 % à 0,5 %), du chlorure de sodium (0,05 % à 0,1 %), du Tween 80 (0,01 % à 0,08 %), MgSO₄·7H₂O (0,01 % à 0,04 %), MnSO₄·4H₂O (0,001 % à 0,01 %) et FeSO₄·7H₂O (0,001 % à 0,008 %), ainsi qu'un extrait à 1 % de *Metschnikowia Pulcherrima* et des combinaisons de ceux-ci, les pourcentages étant exprimés en poids par rapport au poids du bouillon de fermentation.

8. Procédé pour l'obtention de polyamines biogènes selon la revendication 1, **caractérisé en ce que** l'étape iii) consistant à purifier par filtration est mise en oeuvre par l'utilisation de filtres céramiques de 600 nm.

9. Procédé pour l'obtention de polyamines biogènes selon la revendication 1, **caractérisé en ce que** la concentration à l'étape iii) est mise en oeuvre au moyen d'un séchage par pulvérisation, la température de séchage ne dépassant pas 400 °C.

10. Procédé pour l'obtention de polyamines biogènes selon la revendication 1, **caractérisé en ce qu'**il permet d'obtenir un concentré solide de polyamines ayant une teneur comprise entre 2 et 10 %.

11. Procédé pour l'obtention de polyamines biogènes selon la revendication 10, **caractérisé en ce qu'**on obtient un concentré solide qui comprend 0,10 à 1,10 % d'histamine, 0,10 à 1,00 % de cadavérine, 0,10 à 1,00 % de putrescine, 0,10 à 1,30 % de spermidine et 1,60 à 5,60 % d'autres polyamines.
